(19) **Europäisches Patentamt European Patent Office Office européen des brevets**

(11) **EP 3 286 215 B1**

(12) **EUROPEAN PATENT SPECIFICATION**

(45) Date of publication and mention
of the grant of the patent:
**21.10.2020  Bulletin 2020/43**

(51) Int Cl.:
***C07K 16/14*** *(2006.01)*      ***G01N 33/00*** *(2006.01)*
***G01N 33/02*** *(2006.01)*

(21) Application number: **15716583.8**

(22) Date of filing: **24.03.2015**

(86) International application number:
**PCT/IB2015/052150**

(87) International publication number:
**WO 2016/151361 (29.09.2016 Gazette 2016/39)**

(54) **MONOCLONAL IGA ANTIBODY FOR MYCOTOXINS, A HYBRIDOMA CELL LINE PRODUCING SAID ANTIBODY, AND SPECIFIC USE THEREOF IN IMMUNOASSAY**

MONOKLONALER IGA-ANTIKÖRPER FÜR MYKOTOXINE, EINE DIESEN ANTIKÖRPER PRODUZIERENDE HYBRIDOMZELLLINIE UND SPEZIFISCHE VERWENDUNG DAVON IN EINEM IMMUNOASSAY

ANTICORPS IGA MONOCLONAL ANTI-MYCOTOXINES, LIGNÉE CELLULAIRE D'HYBRIDOME PRODUISANT LEDIT ANTICORPS ET UTILISATION SPÉCIFIQUE DE CELUI-CI DANS UN DOSAGE IMMUNOLOGIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**28.02.2018  Bulletin 2018/09**

(73) Proprietor: **Tübitak**
**06100 Ankara (TR)**

(72) Inventors:
• **ÖZTÜRK, Selma**
**41470 Kocaeli (TR)**
• **ERTEKIN, Özlem**
**41470 Kocaeli (TR)**
• **PIRINÇÇI, Serife Seyda**
**41470 Kocaeli (TR)**
• **KOCAAGA, Harun**
**41470 Kocaeli (TR)**

(56) References cited:
**EP-A1- 2 090 590          US-A- 4 818 687**
**US-A1- 2003 203 412**

## Description

### Technical Field of the Invention

The present invention relates to an antibody of IgA class and the use thereof in immunoassays. More particularly the invention relates to the use of an antibody of IgA class in diagnostic systems to detect and/or quantify mycotoxins, especially aflatoxins.

### Background of the Invention

The key features of an antibody being used in analytical systems are affinity, avidity and specifity. Structural character of the antibody is a critical parameter in the design of the immunoassays, It is well known that there are 4 major classes of antibodies secreted from the B cells of mammalian systems; immunoglobulin M (IgM), immunoglobulin G (IgG), immunoglobulin A (IgA) and immunoglobulin E (IgE). Affinity maturation is a process where B cells produce antibodies with increased affinity to the antigen. IgM isotype of antibodies are usually not affinity maturated where other antibody isotypes have higher affinities to the antigen. These antibodies also differ in valency where IgG and IgE are divalent, IgA is tetravalent and IgM is decavalent [Murphy et al., Garland Science New York, NY, USA; 2012]. IgA isotype is a multivalent antibody which experiences affinity maturation towards the antigen. Antibodies of IgG isotype are the most abundant immunoglobulin class in mammals, and in monoclonal antibody studies, they are the most commonly developed ones. With their high affinities, abundance and ease of purification, antibodies of IgG isotype are the most commonly used antibodies in immunoassays. IgM antibodies are commonly used in agglutination studies, yet, their low affinity make them relatively poor choices for immunoassays. However, there are limited number of reports regarding IgA use in antibody based detection systems [US 4,791,067; US 5,534,411]. Previously known anti-aflatoxin antibodies are mostly IgG isotype antibodies [US 20100062542 A1, US 8,153,767 B2, US 20140057294 A1], where limited number of studies utilize IgM class of antibodies [US 4,818,687], Yet, no IgA class monoclonal anti-mycotoxin antibody was reported to be used in analytical systems up to date.

Antibody orientation is a critical factor when antibodies are immobilized to a support since the antigen binding variable regions should be exposed in order to be functional. Typically when antibodies are immobilized on a solid support, less than 30% are correctly oriented and hence, functional. This results in the need for purified antibody solutions for concentrated binding to the immunosorbent surface, a higher amount of the immunosorbent material, and/or a means of orienting the antibody, so that the antigen binding sites are free [US 6,194,552]. The use of multivalent antibodies may be a solution to this problem as there will be free antigen binding sites to be functional even though some are blocked during immobilization. Moreover, the use of multivalent antibodies will allow agglutination reaction, which may be utilized as a means of detection and quantification [US 4,791,067].

Aflatoxins are hepatotoxic mycotoxins that are produced by Aspergilus spp. Aflatoxin B1, B2, G1, G2 are the four common naturally occurring analogs of aflatoxins and aflatoxin M1 is a water-soluble aflatoxin analog found in milk, and produced by animal metabolism. High level exposure to aflatoxins results in acute toxicity which may lead to death, and chronic exposure often leads to liver diseases including liver cancer in humans [Kensler et al., Toxicological Sciences, 2010, 120 (Supplement 1): S28-S48; Shephard, Analytical and bioanalytical chemistry. 2009, 395(5):1215-1224; Robens et al. Reviews of environmental contamination and toxicology. 1992; 127:69].

Aflatoxin levels in food and feedstuff is regulated in many countries due to their toxic effects and several methods are devised to fulfill the requirements of the regulations such as thin layer chromatography, liquid chromatography based methods including high pressure liquid chromatography (HPLC) or liquid chromatography-tandem mass spectrometry (LC-MS/MS); and enzyme based immunological test methods including enzyme linked immunosorbent assay (ELISA) [Shephard, Analytical and bioanalytical chemistry. 2009, 395(5): 1215-1224; Boutrif, Natural toxins, 1995, 3(4):322-326; Commission E. Commission Regulation (EC) No 1525/98 of 16 July 1998, amending Regulation (EC) No 194/97 of 31 January 1997; Official Journal of the European Communities L. 1998;201:436]. Internationally accepted precise aflatoxin quantification methods include instrumental analysis with HPLC or LC-MS/MS and ELISA. Instrumental aflatoxin analysis requires a series of steps including sampling, homogenization, extraction, extract clean-up and detection. The sample clean-up step comprises affinity based purification techniques, particularly immunoaffinity chromatography in order to concentrate and remove the aflatoxins from complex extract matrix [Uchigashima et al., Journal of agricultural and food chemistry, 2009, 57(19):8728-8734]. Both immunoaffinity columns and ELISA systems utilize the ability of an anti-aflatoxin antibody to specifically bind aflatoxins. Accordingly, many antibodies specific to aflatoxins are developed to date.

US 2003/0203412 A1 explains the determination of exposure of humans to fungi or mycotoxins by measuring the level of IgA production by exposed person's immune system. This patent differs from the present invention in two ways. First, US 2003/0203412 A1 does explain the detection or quantification of mycotoxins, it relates with the detection of patient exposure to mycotoxins unlike our proposal which proposes the use of an antibody of IgA class for detecting and quantifying mycotoxins. Secondly, the analyte to be determined is not mycotoxins, but IgA itself. IgA molecules are not specifically developed for the detection of exposure, they are the natural result of patient's exposure. US 4,818,687 is also related with exposure detection. However, in this case, a monoclonal IgM antibody is provided, which is used to detect exposure of humans and animals to aflatoxins. So, the document differs from our patent proposal both in terms of antibody isotype, and the aim of the invention.

EP 2 090 590 A1 states the usage of IgG1 class antibody which requires antibody orientation during immobilization at the sensing surface. IgA class antibody in the present invention is more tolerant to low pH values down to pH:4. Also, no matrix interference is tested in this document in comparison to the present invention which includes to test matrix interference effects with corn, red pepper and hazelnut extracts.

## Disclosure of the Invention

The present invention relates to an antibody of IgA class and the use thereof in immunoassays including diagnostic systems. Preferably, the present invention relates to the use of an antibody of IgA class in diagnostic systems to detect and/or quantify mycotoxins, particularly aflatoxins.

## Technical Problem

Antibodies against aflatoxins are conventionally required to measure 4 naturally occurring aflatoxin isotypes and one water soluble metabolite in food and feedstuff. The methods employed require the antibody to be immobilized on an immunosorbent surface and functionally interact with aflatoxins, on the surface, in organic solvent extracts of samples to be analyzed. Bivalent antibodies used up to date are disadvantageous in that, for full antibody activity, the antibody must be oriented before immobilization in order to compensate the activity loss resulting from the steric hindrance during immobilization. Yet, the objective of the present invention is to develop novel murine monoclonal antibodies of IgA class which do not necessitate the orientation prior to immobilization on an immunosorbent surface.

Most of the antibodies require to be purified prior to use. Purification step both increases the production costs and affects the activity of the antibody. Another object of this invention is to provide an antibody that can be used without purification after the concentration of the antibody.

An antibody specific to aflatoxin can be used in immunoanalytical systems in order to detect and/or quantify aflatoxin contamination in food and feedstuff. Aflatoxin analysis is often conducted with a liquid extract of the sample to be analyzed. Preparation of the liquid extracts of solid samples is achieved by the use of organic solvents. During the extraction process, several metabolites of the sample are co-extracted with the aflatoxins. For an antibody to be effectively utilized in aflatoxin analysis, its solvent tolerance and extract cross-reactivity should be well established. Furthermore, alternative extraction protocols with high ionic strength or acidic buffers may be employed in order to increase the extraction yield. pH and ionic strength variations in buffers may also be used to increase the specifity of the antibody by preventing binding of non-aflatoxin extract components. Therefore, another objective of the current invention is to provide antibodies which display the desired solvent tolerance, acid and salt stability and do not have cross reactivity to other toxins.

## Technical Solution

The present invention relates to an antibody of IgA class and the use thereof in immunoassays including diagnostic systems. Preferably, the present invention relates to the use of an antibody of IgA class in diagnostic systems to detect and/or quantify mycotoxins, particularly aflatoxins.

In another aspect, the present invention relates to a novel murine monoclonal antibody of IgA class or a fragment thereof which has high affinity to aflatoxin B1, B2, G1, G2 and M1 and can be used in immunoassays for aflatoxin detection.

Accordingly, an objective of the present invention is to provide an IgA isotype murine monoclonal antibody, which has affinity towards aflatoxin isotypes B1, B2, G1, G2 and M1, yet does not display cross reactivity to other toxins. Another aim of the current invention is to develop such an antibody which can be tolerant to organic solvents, acidic pH and high ionic strength solutions used during the purification of the antibody with different extraction methods and/or the diagnostics immunoassays on different matrices which are found as liquid extracts of the sample to be analyzed. In this regard, another objective of the present invention is to develop a diagnostic system which involves the use of antibodies disclosed.

Another objective of the present invention is to develop a hybridoma clone capable of producing the antibody of IgA class of the current invention. In this respect, one of the aims of the invention is to present a method of growing the hybridoma clone producing an antibody of IgA class in cell culture.

The antibody developed further needs to be purified from the cell culture supernatant. To this end, an extraction of the antibody from the cell medium is required. In this regard, another objective of the current invention is providing a method to purify the antibody from the cell culture supernatant

In another perspective, the current invention provides novel use of the antibodies according to the instant invention in immunoassays. Said immunoassays include but are not limited to immunoaffinity column, enzyme immunoassay or immuno-chromatographic strip assay, agglutination tests and immunobiosensor development.

## Description of the Drawings

Figure 1 illustrates the mycotoxin inhibition assay showing the specifity of D12E2 antibody.

Figure 2 illustrates the DEAE ion exchange chromatogram for the purification of D12E2 antibody.

Figure 3A illustrates the silver stained, reduced, Sodium Dodecyl Sulfate Polyacrylamide Gel Electrophoresis (SDS PAGE) gel loaded as discussed in the Examples Lane 1: SDS PAGE marker (thermo Scientific 26616), Lane 2; 0.2 $\mu$g BSA (Sigma, B2518), Lane 3; 3.5 $\mu$g protein sample from the first peak of the chromatography, Lane 4: 0.5 $\mu$g protein sample from the second peak of the chromatography.

Figure 3B illustrates the western blot and immunoblot analysis of the similarly loaded SDS PAGE gel as discussed in the Examples. Lane 1: SDS PAGE marker (thermo Scientific 26616), Lane 2: 0.2 $\mu$g BSA (Sigma, B2518), Lane 3: 3.5 $\mu$g protein sample from the first peak of the chromatography, Lane 4: 0.5 $\mu$g protein sample from the second peak of the chromatography.

Figure 4 Illustrates the tolerance of D12E2 antibody to aqueous solutions of Methanol, Acetonitrile, Acetone and Ethanol at varying concentrations.

Figure 5 illustrates the pH stability of the D12E2 antibody.

Figure 6 illustrates the salt stability of D12E2 antibody.

Figure 7 illustrates the aflatoxin B1 inhibition curve of D12E2 antibody in the presence of Hazelnut, Red pepper and Corn extracts.

Figure 8 illustrates the standard curves of ELISA test

## Detailed Description of the Invention

The present invention relates to an antibody of IgA class and the use thereof in immunoassays including diagnostic systems. In a preferred embodiment, the present invention relates to the use of an antibody of IgA class in diagnostic systems to detect and/or quantify mycotoxins, particularly aflatoxins.

## Advantageous Effects

The present invention also relates to monoclonal IgA antibodies specific for aflatoxins B1, B2, G1, G2 and M1. The antibody of the invention is characterized by its isotype IgA which is tetravalent and effectively binds to the aflatoxin in different matrices such as corn, red pepper and hazelnut extracts without need for further purification steps. The antibody can effectively be used in immunoaffinity column, ELISA kit and biosensor development. In the immunoaffinity column application, the antibody disclosed in the current invention displayed high aflatoxin recovery rates when 5 ng of each aflatoxin where retention rates were 104.9±5.4% for aflatoxin B1, 82.4±4.8% for aflatoxin B2, 85.5±6.9% for aflatoxin G1 and 70.7±7.1% for aflatoxin G2.

The tolerance of the antibody developed to different solvents is extremely high for the use thereof in aflatoxin detection and the tolerance levels fulfill the requirements for international analytical standards. Moreover, the stability of the antibody should be maintained in acidic conditions where the interaction of the antibody-antigen interaction is not disrupted. The stability is useful in order to obtain high purity antibody by affinity chromatography where aflatoxin-protein conjugate is immobilized to the affinity column and will also provide insight for the regeneration of active biosensor surfaces after analysis. Hence, the antibody of the invention is characterized by its tolerance to 40% (w/w) methanol, 20% (w/w) acetonitrile, 30% (w/w) acetone and 40% (w/w) ethanol; and its stability at 1000 mM NaCl and acidic pH down to pH 4.

The present invention further provides a hybridoma clone capable of producing the antibody of the current invention and a method for growing said clone in a cell culture. According to another aspect of the present invention there is disclosed a method for purifying the antibody from the cell culture supernatant of the hybridoma cell line.

In another aspect, the current invention provides the use of antibodies of the invention in immunoassays including but not limited to immunoaffinity column, enzyme immunoassay or immuno-chromatographic strip assay, agglutination tests and immunobiosensor development.

In this respect, the present invention is related to a method of immunologically detecting aflatoxins wherein said method comprises an enzyme immunoassay developed by using the antibody or a fragment thereof of the invention.

The present invention further discloses a method to quantify and/or detect aflatoxins by using immunobiosensors developed with an antibody of the invention immobilized on a recognition surface for use in methods including but not limited to electrochemical, optical, calorimetric, mass sensitive (piezoelectric/acoustic) or magnetic transducer.

The antibody disclosed in the present invention does not require further purification steps after the cell culture supernatant is concentrated through ammonium sulfate precipitation method when the antibody will be used in an immunoaffinity column. On the other hand, if an ELISA test is desired to be performed with the antibody of the invention, the invention provides a simplified purification method of the antibody from hybridoma cell culture supernatant which is characterized by the use of DEAE ionexchange chromatography method with at least 80% purity.

More specifically, the present invention discloses an IgA class monoclonal D12E2 antibody specific to aflatoxin B1, B2, G1, G2 and M1 where said antibody comprises a heavy chain polypeptide having the sequence of SEQ ID NO:1 and a light chain polypeptide having the sequence of SEQ ID NO: 2.

The present invention will be described in detail in connection with specific embodiments and different aspects with reference to the accompanying drawings. Unless the context requires otherwise, throughout the following specification and claims, the word "aflatoxin" refers to at least one or all of Aflatoxin B1, B2, G1, G2 and M1; and the word "D12E2 antibody" refers to the IgA type monoclonal antibody produced by the hybridoma clone D12E2 or a fragment thereof comprising SEQ ID NO:1 and SEQ ID NO: 2.

The term "immunoanalytical methods" refers to conventional immunoanalytical methods which are well known in the state of art that include immunoaffinity chromatography, ELISA, immunobiosensors etc.

The term "organic solvent tolerance" points the ability of the antibody to bind to its antigen at a specified concentration of methanol, ethanol, acetonitrile or acetone for a specified duration without losing more than 70% of its activity.

The term "antibody cross reactivity" refers to the affinity of the antibody to antigens other than aflatoxins including but not limited to other mycotoxins and food extracts.

Hereinafter, the present invention is described in more detail with reference to the Examples, which are not intended to limit the technical scope of the present invention.

## A. Development of the Antibody

The method for development of the anti-aflatoxin antibody comprises the following steps:

### 1. Preparation of aflatoxin conjugates

Development of murine monoclonal antibodies initially requires a strong humoral immune response from the mice. Aflatoxins are haptens which are too small to be immunogenic, and they need to be conjugated to immunogenic carrier molecules in order to evoke a hapten specific antibody response. The conjugation of the aflatoxins to designated carriers is also necessary for the immobilization of the haptens to a solid support in immunoassays including but not limited to indirect ELISA, competitive indirect ELISA or biosensor tests. The hapten used for producing the disclosed antibody was Aflatoxin B1 or derivative thereof. The term "carrier" used herein includes natural proteins of non-murine origin. The aflatoxin B1 conjugates implicated in this invention includes Aflatoxin B1 - human apotransferrin (hTF) and aflatoxin B1-ovalbumin (OVA) which were utilized as aflatoxin immunogen in mice immunizations, and Aflatoxin B1-Bovine Serum Albumin (BSA) which was utilized as the solid phase antigen to be used in indirect and competitive indirect ELISA tests.

Aflatoxin-protein conjugation was achieved by mannich type reaction. For this reaction, the protein carrier was first modified with ethylenediamine so that the amine groups of the protein were enriched. The amine groups of resulting cationized proteins were condensed with formaldehyde and the $\alpha$-hydrogen adjacent carbonyl in aflatoxins with the modification of the method of Zhou et al., 2007. Aflatoxin-protein conjugates were stored in small aliquots at -20°C.

### 2. Immunization

Balb/c mice were intraperitonally immunized with 100 μg of Aflatoxin B1-hTF conjugates. In the first dose of injection, complete Freund's adjuvant was used. The subsequent doses were prepared with incomplete Freund's adjuvant. Antibody response of the mice was initially evaluated by indirect ELISA with aflatoxin B1-BSA coated wells. In order to ensure that antibodies produced by mice, which is immunized with protein conjugated aflatoxin, were capable of binding free aflatoxins, competitive indirect ELISA with Aflatoxin B1 was performed. 5 doses of the conjugate were injected to mice until the desired antibody response was achieved. A booster immunization with 50 $\mu$g of aflatoxin B1-OVA conjugate was intravenously injected to the mouse with highest aflatoxin specific antibody response.

3. Cell fusion and selection

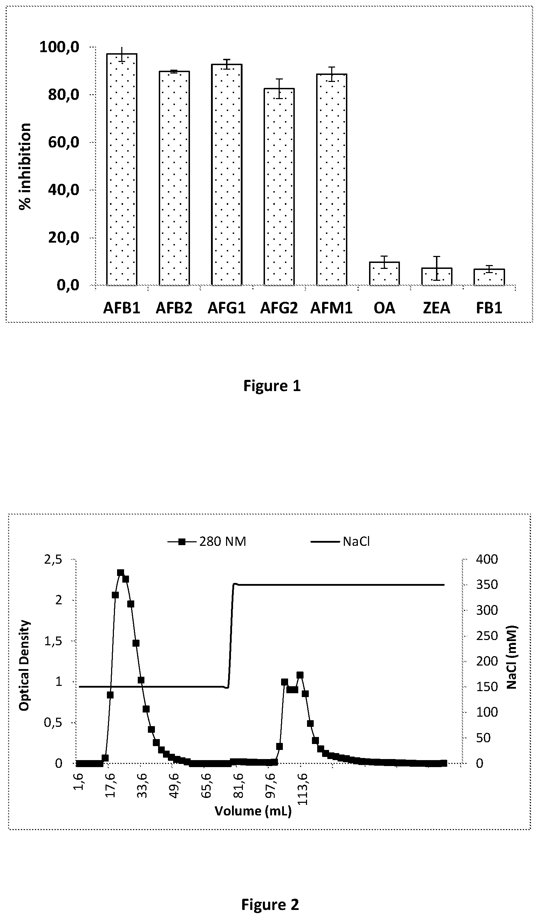

The spleen of the immune responsive mice was used as the source of B lymphocytes capable of antibody production against aflatoxins. In vitro lifespan of B lymphocytes is limited, therefore it is not possible to obtain sufficient quantity of antibody molecules to be used in immunoassays. However, these cells can be immortalized by their fusion with plasma cell derived from myeloma cells with hybridoma method. The immortalized cells were screened and selected for their anti-aflatoxin antibody production performance by indirect ELISA with AFB1-BSA coated plates. The antibody producing positive clones were selected and transferred to 24 well culture plates so that culture supernatants will be collected for further testing. Supernatants of the antibody producing hybridoma clones were tested for their cross reactivity with the proteins used in immunizations by indirect ELISA. Clones and subclones tested negative for protein cross reactivity were further evaluated for aflatoxin specifity by mycotoxin inhibition assay. The immunizations were done with immobilized aflatoxin B1, so the initial mycotoxin inhibition assay aimed to test the reactivity of the antibodies with free aflatoxins. The clones producing the antibodies inhibited by free aflatoxin B1, B2, G1, G2 and M1 were further tested for binding to other mycotoxins such as ochratoxin A, Zearelanone and Fumonisin B1 in order to assess specifity. Aflatoxin specific clones were selected and their isotypes were determined with commercially available mouse monoclonal subisotyping kits according to the manufacturer's instructions. Clones producing IgA isotype antibodies were subcloned to monoclonal cell lines. The cells were cloned by limiting dilution and single clones were selected by indirect ELISA. The cloning and selection phase was repeated 3 times until stable hybridoma cell line D12E2 was obtained. The mycotoxin inhibition assay results of D12E2 antibody is given in Figure 1.

**B. Preparation and Characterization of the D12E2 monoclonal antibody**

D12E2 antibody was obtained from the cell culture supernatants of D12E2 hybridoma cell line. The culture conditions of the stable IgA producing hybridoma cell line D12E2 was optimized so that D12E2 cells were grown in 5% FBS containing Dulbecco's Modified Eagle Medium (DMEM) and produce functional antibodies. D12E2 cells were frozen and thawed retaining their antibody production capacity. Frozen cells were stored at -150°C refrigerated freezer. A parallel stock was kept in liquid nitrogen.

Culture supernatants were partially purified and 20 fold concentrated by ammonium sulfate precipitation. The use of the antibody without a need for further purification will significantly reduce the production costs of test systems. So, all the characterization studies were conducted with ammonium sulfate precipitated antibody solution. Either ammonium sulfate precipitates or purified samples of the D12E2 antibody can be used for immunoanalytical uses of the antibody. The antibody was used in immunoaffinity columns without further purification. One step purification method by DEAE ion exchange chromatography, which results in 80% purified antibody, was optimized for the antibody to be used in ELISA test system.

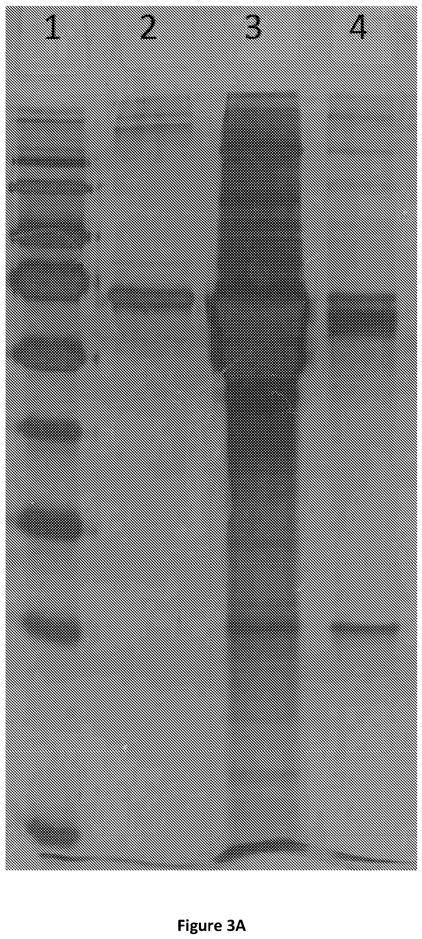

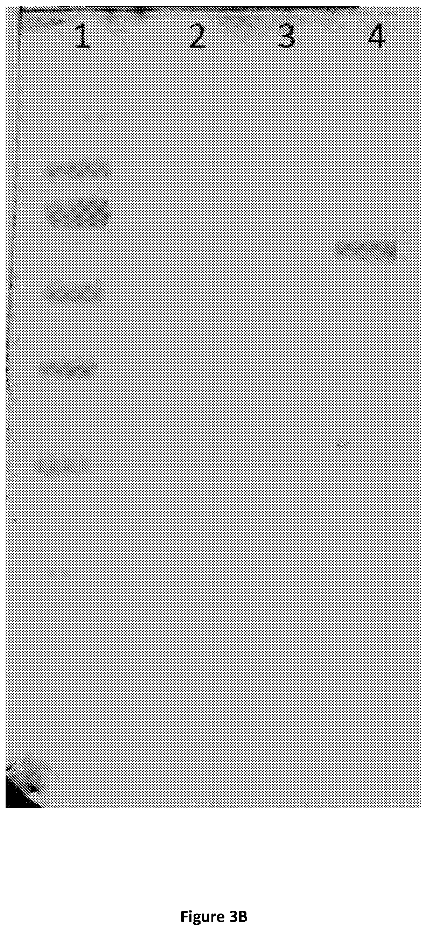

DEAE Cellulose column was used for the purification of the antibody by modified standard protocols (Bruck et al., Journal of Immunological Methods, 1982, 53: 313-319). Ammonium sulfate precipitated antibody was loaded to the column at pH 7 with 150 mM NaCl where most of the serum proteins were eluted and antibody was bound to the column. The bound antibody was eluted at the same buffer with 350 mM NaCl concentration. Figure 2 shows the chromatogram of the purification procedure. The resulting two peaks were analyzed by SDS PAGE and western blot which is followed by an immunoblot aiming to visualize the antibodies transferred to the membrane. In the SDS PAGE and Western blot analysis, 3,5 $\mu$g protein was loaded from the first peak and 0,5 $\mu$g of protein was loaded from the second peak to their respective wells in order to prove the absence of the antibody in the first peak with immunoblot. SDS PAGE analysis disclosed in Figure 3-A shows the exact same amount of protein that were transferred to the PVDF membrane in immunoblot analysis disclosed in Figure 3-B. The immunoblot analysis showed that; despite the significant difference in the amount of protein loaded; antibody signal was only observed in the second peak, seen in lane 4 of the western blot membrane, indicating that the antibody was concentrated in the second peak. The analysis of SDS PAGE indicated 80% purity of the antibody by one step purification procedure after the ammonium sulphate precipitation.

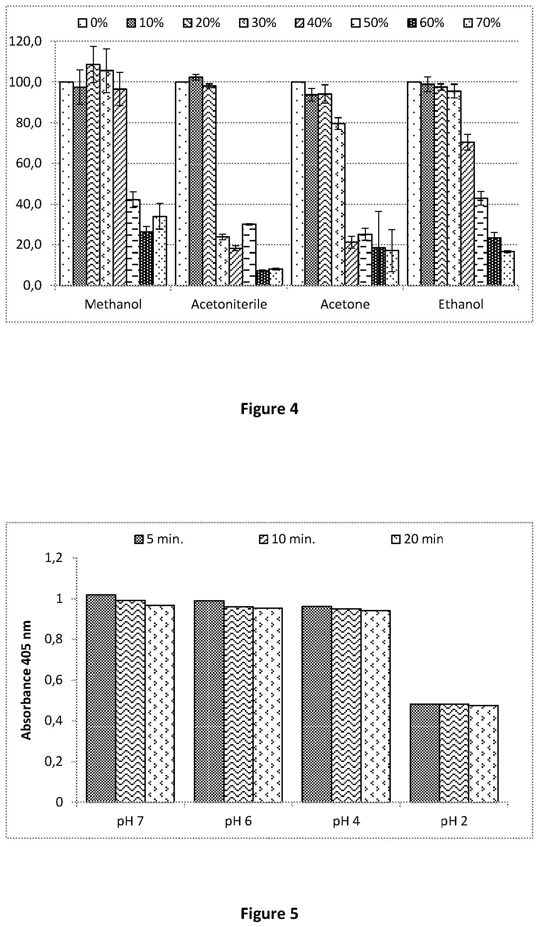

D12E2 antibody was developed for use in immunoanalytical systems in order to detect and/or quantify aflatoxin contamination in food and feedstuff. The solvent tolerance of the antibody was assessed by indirect ELISA test. Methanol, acetonitrile, acetone and ethanol stabilities of D12E2 antibody were examined. Antibody was diluted in 0%, 10%, 20%, 30%, 40%, 50%, 60% and 70% aqueous solutions of methanol, acetonitrile, acetone and ethanol. Indirect ELISA was conducted with antibodies diluted in solvents on aflatoxin B1-BSA coated wells. The results showing the binding capability of the D12E2 antibody to aflatoxin B1-BSA conjugate in solutions with increasing concentrations of solvents are disclosed in Figure 4. D12E2 antibody retained full activity in 40% methanol solution. The antibody showed high tolerance to acetonitrile where no activity loss was observed in 20% acetonitrile solution. D12E2 antibody did not lose its activity at 20% acetone solution and retained 80% activity at 30% aqueous solution of acetone. Ethanol did not affect the activity of D12E2 antibody up to 30% concentration and was 70% active in 40% aqueous solution of ethanol. Hence, D12E2 antibody was shown to be tolerant to 40% Methanol, 20% acetonitrile, 30% Acetone and 40% Ethanol.

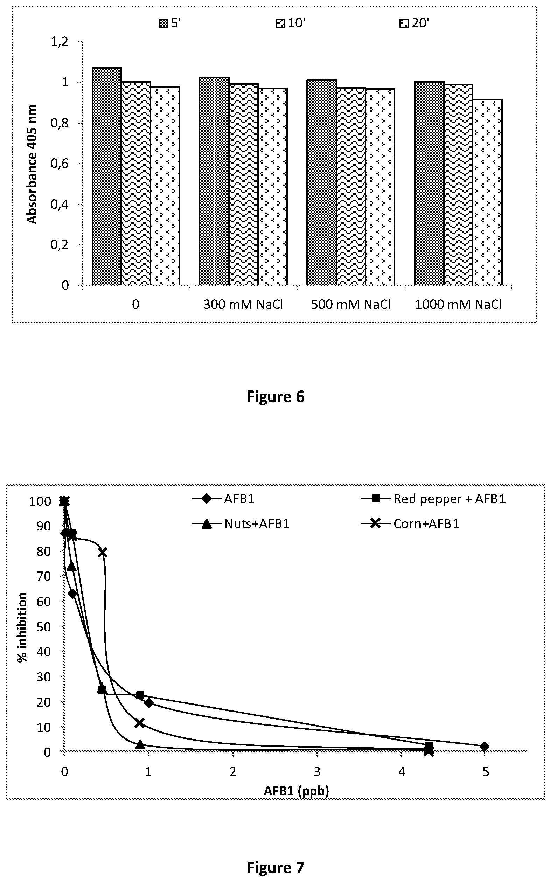

The acid and salt stability of the D12E2 antibody binding to aflatoxin B1 was assessed by indirect ELISA. The antibody was incubated on aflatoxin B1-BSA coated ELISA plates. The bound antibody was exposed to buffered solutions of different pH and salt concentrations for 5, 10 and 20 minutes. The wells were washed and remaining bound antibody was visualized by indirect ELISA. The results showing the pH stability of the antibody at pH 7, 6,4 and 2 are presented in Figure 5. According to these results, D12E2 antibody-aflatoxin binding is stable down to pH 4, At pH 2, 50% of the bound antibody was disassociated from the aflatoxin. Salt stability of the antibody at 0, 300 mM, 500 mM and 1000 mM NaCl concentrations at neutral pH are presented in Figure 6. The results showed that the antigen-antibody interaction was stable at all applied concentrations, including 1000 mM NaCl.

In order to assess the matrix interference effect to the D12E2 antibody binding to aflatoxin B1; corn, red pepper and hazelnut extracts were evaluated. The binding inhibition curves of D12E2 antibody with aflatoxin B1 in PBS and in the presence of food extracts were compared. Clean corn, red pepper and hazelnut extracts were spiked with aflatoxin B1. The spiked extracts and aflatoxin B1 spiked PBS, were preincubated with D12E2 antibody and matrix effects are evaluated by indirect competitive ELISA, The resulting binding inhibition curves are presented in Figure 7. The results showed that D12E2 antibody effectively binds to the aflatoxin B1 in different matrices.

The result obtained from the characterization of the D12E2 antibody showed that the antibody was a good candidate to be used in immunoanalytical systems. Immunoaffinity column, ELISA and biosensor applications of D12E2 antibody was disclosed as a part of this invention with examples which proved the effectiveness of the antibody in immunoanalytical systems.

## C. Development of aflatoxin immunoaffinity column with D12E2 antibody

Immunoaffinity columns can be prepared by conjugating the antibody to a solid phase support capable of binding a protein molecule via its reactive moieties. The solid phase support used in the context of this invention to exemplify the use of the antibody in immunoaffinity column systems is cyanogen bromide (CnBr) activated sepharose. Immunoaffinity columns were prepared by conjugating ammonium sulphate precipitated D12E2 antibody solution containing 60 µg antibody to CnBr activated sepharose with the methods obvious to the skilled in the art. There was no need for orienting or further purifying the antibody. The resulting immunoaffinity columns were evaluated with two parameters; aflatoxin binding capacity and the ability to bind limit amount of aflatoxins.

Aflatoxin binding capacity of the columns was evaluated by overflow test. 500 ng of aflatoxin B1, aflatoxin B2, aflatoxin G1 and aflatoxin G2 in 20 mL 20% methanol-water were loaded to the columns in separate occasions and total binding capacity of the columns were calculated by HPLC analysis for each aflatoxin isotype. The overflow test results showed that the immunoaffinity columns prepared by partially purified D12E2 antibody were able to bind $111 \pm 6.5$ ng aflatoxin B1, $70 \pm 4.9$ ng aflatoxin B2, $114 \pm 5.8$ ng aflatoxin G1 and $73 \pm 2.4$ ng aflatoxin G2. The test was repeated with corn extract. 34-41% reduction in total aflatoxin binding capacity was observed for aflatoxins B1, G1 and G2 and 18% reduction was observed for aflatoxin B2.

Limit binding test was conducted using 16% methanol-water solution contaminated with a mixture of aflatoxin B1, aflatoxin B2, aflatoxin G1 and aflatoxin G2 so that 5 ng of each toxin was loaded to the columns. The percentage of the recovered aflatoxins was used for evaluation. The limit binding test results showed that the binding performance of the immunoaffinity columns prepared by partially purified D12E2 antibody were $104.9 \pm 5.4\%$ for aflatoxin B1, $82.4 \pm 4.8\%$ for aflatoxin B2, $85.5 \pm 6.9\%$ for aflatoxin G1 and $70.7 \pm 7.1\%$ for aflatoxin G2.

## D. Development of aflatoxin ELISA test with D12E2 antibody

ELISA test system based on direct competitive ELISA for rapid, easy and on-site detection and quantitation of aflatoxin B1 was developed by the use of purified D12E2 antibody. The designed test system involves the immobilization of D12E2 antibody to the ELISA plate without any need for orientation. Quantitation was based on the competition of enzyme labeled aflatoxin B1-protein conjugate with different concentrations of aflatoxin in the solution. Aflatoxin B1-protein conjugate was labeled with HRP with the use of gluteraldehyde method. The test enables the quantification of aflatoxin B1 in corn, red pepper and hazelnut extracts. Detection range of the kit in aflatoxin B1 spiked extracts is defined as 1-50 ppb. Detection between these limits allows control of all foods and feeds except baby foods and cereals intended for infants which requires detection below 0.1 ppb. Reduction of measured enzyme activity due to 50 ppb free aflatoxin B1 competition in the presence of %25 corn, red pepper and hazelnut extracts are respectively 68%, 81.5% and 73%.

**E. Best Mode for Carrying out the Invention**

**Example 1: Preparation of cationized proteins**

Bovine serum albumin (BSA), ovalbumin (OVA) and human apotransferrin (hTF) were cationized in the preparation of immunogens for antibody development. 670 μL of ethylenediamine (EDA) was added to 5 mL of MES buffer (0.1 M, pH:4.8). The pH of the solution was adjusted to 4.8 with HCl. 25 mg protein was dissolved in 5 mL EDA solution. Cationization reaction was started with the addition of 18 mg 1-Ethyl-3-[3-dimethylaminopropyl]-carbodiimide hydrochloride (EDC). Resulting solution was stirred for 1 hour at room temperature. Reaction was stopped by the addition of 150 μL sodium acetate (4M, pH:4.8). The unreacted EDA and EDC was removed by dialysis against distilled water. Cationization efficiency was tested with HPLC using strong cation exchange column (Agilent Bio IEX SCX NP5). The proteins were loaded to the column at neutral pH with 50 mM Tris-HCl pH: 7 where cationized proteins were retained in the column and unreacted proteins were not. The retained cationized proteins were eluted at higher pH and salt with 50 mM Tris-HCl pH: 10.4, 1 M NaCl. The proteins were detected with florescent dedector (ex:280 nm, em: 350 nm).The column was regenerated with neutral buffer (50 mM Tris-HCl). Cationization efficiency was found to be higher than 95%. Resulting cationized proteins were lyophilized and stored at -20 °C until used.

**Example 2: Preparation of aflatoxin conjugates**

The conjugate between aflatoxin B1 and the cationized proteins prepared in example 1 was achieved with mannich type reaction with the method modified from Zhouet al., Journal of Immunological Methods, 2007, 328(1-2), 79-88. 500 μg of cationized protein was dissolved in 753 μL MES buffer (0.1M pH 4.8). 47 μL 2 mg/mL aflatoxinB1 in dimethylformamide (DMF) and 50 μL formaldehyde were added to the protein solution. The resulting solution was mixed for 24 hours at 37°C. Aflatoxin B1-protein conjugate was purified at least five times by 10 kDa MWCO centrifugal filter devices. Conjugation efficiency was evaluated spectroscopically based on Beer-Lambert law; with the following formula.

$$C(\text{aflatoxin})/C(\text{BSA}) = A_{360} \times \varepsilon P_{280} /(A_{280} \times \varepsilon \text{aflatoxinB1}_{360} - \varepsilon \text{aflatoxinB1}_{280} \times A_{360})$$

Where, $A_{360}$: Absorbance of the conjugate at 360 nm, $A_{280}$: Absorbance of the conjugate at 280 nm, $\varepsilon P_{280}$: Extinction coefficient of cationized protein at 280 nm, $\varepsilon \text{aflatoxinB1}_{360}$: Extinction coefficient of aflatoxin B1at 360 nm, $\varepsilon \text{aflatoxinB1}_{280}$: Extinction coefficient of aflatoxin B1at 280 nm.

Aflatoxin-protein conjugates were stored at -20°C in small aliquotes until used in mouse immunization and plate coating for ELISA.

**Example 3: Immunization of mice**

3 female 8 weeks old Balb/c mice were intraperitonally immunized with 100 μg of Aflatoxin B1-hTF conjugates. The first dose of injection was prepared by emulsifying Aflatoxin B1-hTF solution with an equal volume of complete Freund's adjuvant. Subsequent immunizations were prepared by mixing the antigen solution with equal volume of incomplete Freund's adjuvant. The mice were immunized 5 times at days 0, 14, 36, 56 and 78. The mice were bled at day 12 after each immunization and sera were collected by centrifugation. Anti-aflatoxin antibody titer was evaluated with indirect ELISA. Aflatoxin B1-BSA conjugate was coated to ELISA plates for analysis so that only the antibody response against aflatoxin B1 would be observed without the interference of anti-hTF antibodies. The sera of the mice with anti-aflatoxin immune response were tested with Aflatoxin B1 inhibition assay in order to confirm the reactivity of the sera with free aflatoxin B1. The mouse with the strongest anti-aflatoxin immune response was selected and received an intravenous booster immunization with 50 μg aflatoxin B1-OVA conjugate 3 days prior to fusion so that B cells producing aflatoxin specific antibodies would selectively be enriched.

**Example 4: Control of immunizations with Indirect ELISA Method**

Aflatoxin B1-BSA conjugate was used as the detection antigen in indirect ELISA. Antigen was diluted to 5 μg/mL in phosphate buffered saline (PBS, 10mM PO4, 150 mM NaCl, pH:7.2) and 100 μL of the resulting solution was dispensed to each well of a 96 well ELISA plate to coat the wells with 500 ng of antigen. Some wells were incubated with PBS as negative control. The plate was incubated overnight at +4°C. The wells were washed three times with % 0.2 Tween-PBS and blocked with 200 μL 1% skimmed milk solution in PBS for 1 hour at room temperature. Wells were washed three times with % 0.2 Tween-PBS. Mice sera were diluted 10.000 fold with PBS and 100 μL of the diluted sera was dispensed to the designated wells. Non-immunized mousesera was similarly diluted and used as negative control.

After 1 hour of incubation at room temperature, wells were washed three times. Alkalene phosphatase (AP) conjugated rabbit anti mouse polyvalent antibody developed against mouse IgG, IgM and IgA was 2.000 fold diluted with PBS and dispensed 100 $\mu$L/well. ELISA plate was incubated at 37°C for 1 hour and wells were washed 5 times with % 0.2 Tween-PBS. AP substrate 4-nitrophenyl phosphate was dissolved in substrate buffer (1mM ZnCl2, 1mM MgCl2, 0.1M glycine, pH 10.4) with 1 mg/mL concentration and 100$\mu$L/well was dispensed and incubated for 60 min. at room temperature. Absorbance at 405 nm was measured with a microplate reader.

**Example 5: Control of immunizations with aflatoxin Inhibition assay**

ELISA plates were coated with 500 ng Aflatoxin B1-BSA conjugate and blocked with 1 % skimmed milk solution as explained. 20 $\mu$g/mL aflatoxinB1 solution in PBS was prepared. 50 $\mu$L 50 fold diluted mice sera was mixed with 50 $\mu$L aflatoxin B1 solution containing 1 $\mu$g aflatoxin B1 and incubated for60 min. at 37°C. Similarly diluted mousesera mixed with PBS was used as negative control. 100 $\mu$L of the preincubated sera was dispensed to the wells. After 1 hour of incubation at room temperature, wells were washed thoroughly as stated before. 2000 fold PBS diluted AP conjugated rabbit anti mouse polyvalent antibody was dispensed 100 $\mu$L/well. ELISA plate was incubated at 37°C for 1 hour and wells were washed. 100$\mu$L of 1mg/mL AP substrate 4-nitrophenyl phosphate dissolved in substrate buffer was added to the wells and incubated for 60 min. at room temperature. Absorbance at 405 nm was measured with a microplate reader.

**Example 6: Development of monoclonal antibody producing hybridoma cell line**

Murine myeloma cell line F0 was used for the hybridoma production. Frozen stock of F0 cell line was recovered 10 days prior to the fusion and cultured for 3-4 passages so that 2-3x10$^8$ cells which are in the logarithmic phase of their growth with more than 90% viability are obtained on the day of fusion. 10 culture plates to be used for the dispersion of cells after the fusion was layered with feeder cells the day prior to fusion to provide support to the newly fused hybridoma cells. Feeder cells were prepared from the peritonal cells of a non-immunized Balb/C mouse which was sacrificed by cervical dislocation. The mouse selected for its high aflatoxin specific antibody titer received a booster immunization three days prior to fusion as explained in example 3. On the day of fusion, the immunized mouse was sacrificed with cervical dislocation. Spleen cells were isolated from the mouse and myeloma cells were harvested from the culture plates. Cells were washed three times with 30 mL PBS. The spleen cells and F0 cells were mixed with 1:3 F0:spleen cell ratio. The fusion was achieved by the addition of 1 mL 50% PEG 4000 solution to the cells at 37°C. The fused cells were suspended by subsequent addition of serum free DMEM and DMEM containing 15% Fetal Bovine Serum. After one hour incubation, cells were collected by centrifugation, re-suspended in DMEM containing 20% Fetal Bovine Serum and %2 HAT (hypoxanthine-aminopterin-thymidine) and dispensed to the feeder inoculated culture plates. 10 days after the fusion, the hybrid clones were screened for the production of aflatoxin specific antibody production with indirect ELISA method explained in example 4 where culture supernatant replaced the diluted mouse sera. Selected clones were tested for aflatoxin specifity with indirect ELISA method disclosed in example 4 by replacing the coating antigen with 100 ng BSA, hTF or OVA and immune sera with undiluted cell culture supernatant. Clones showing no cross reactivity with the specified proteins were tested for aflatoxin reactivity with mycotoxin inhibition assay.

**Example 7: Determination of antibody specifity with mycotoxin Inhibition assay**

ELISA plates were coated with 500 ng Aflatoxin B1-BSA conjugate and blocked with 1 % skimmed milk solution as explained in example 4. 20 $\mu$g/mL stock of the Aflatoxins B1, B2, G1, G2, M1, ochratoxin A, zearelanone and Fumonisin B1 was prepared in PBS. 50 $\mu$L cell culture supernatant was mixed with 50 $\mu$L each specified mycotoxin solution containing 1 $\mu$g of mycotoxin separately and incubated 30 min, at 37°C. Cell culture supernatants mixed with PBS was used as negative control. 100 $\mu$L of the preincubated sera was dispensed to the wells. After 1 hour of incubation at room temperature, wells were washed thoroughly as stated before. 2000 fold PBS diluted AP conjugated rabbit anti mouse polyvalent antibody was dispensed 100 $\mu$L/well. ELISA plate was incubated at 37°C for 1 hour and wells were washed. 100$\mu$L of 1mg/mL AP substrate 4-nitrophenyl phosphate dissolved in substrate buffer was added to the wells and incubated for 60 min. at room temperature. Absorbance at 405 nm was measured with a microplate reader.

**Example 8: Production of D12E2 antibody**

D12E2 cell line was cultured in DMEM supplemented with 5% fetal bovine serum and 0.1% Gentamycin at 37°C in the presence of 5% $CO_2$ with 90% humidity. The cell line retained the antibody production capability even at 3% fetal bovine serum supplement, however, cell viability was significantly reduced. The cells were observed to be suspended during culture in 300 cm$^2$ flasks.

D12E2 cells were successfully frozen and regenerated. In order to prepare the frozen stocks of the cells, 2-3x10$^6$

cells were harvested from cell culture flasks and were suspended in 1 mL freezing medium (10% Dimethylsolfoxide (DMSO), 20% fetal bovine serum, 70% DMEM). Cryovial containing the cells were gradually cooled to -150°C and stored either at -150°C refrigerated freezer or at liquid nitrogen.

Frozen cells were thawed by rapid warming at 37°C. Thawed cells were washed twice in PBS and transferred to 25 cm$^2$ flasks in 5 mL 10% fetal bovine serum containing DMEM. Cells were transferred to the previously defined culture medium after one passage.

Cell culture supernatants were partially purified by ammonium sulfate precipitation. In this method, collected supernatants were mixed with equal volume of saturated ammonium sulfate and mixed overnight at +4°C. The precipitate containing the antibody were collected by centrifugation and dissolved in PBS. The antibody solution was exhaustively dialyzed against PBS. The procedure yielded 20 times concentration of the antibody relative to cell culture supernatant. D12E2 ammonium sulfate precipitates were stored at -20°C in 2 mL aliquots until used.

D12E2 antibody was purified by ion exchange chromatography with the use of DEAE cellulose matrix. 20 mL DEAE cellulose column was prepared according the suppliers recommendations and washed with loading buffer (10 mM KPO$_4$, 150 mM NaCl, pH:7). 2 mL Ammonium sulfate precipitated D12E2 antibody was loaded to the chromatography column. Column was washed with 2-3 column volumes of loading buffer and the bound D12E2 antibody was eluted with 10 mM KPO$_4$, 350 mM NaCl, pH:7. Flow rate during the procedure was 30-40 mL/h. 1.5 mL fractions were collected during chromatography and protein elution was monitored by measuring the absorbance at 280 nm. Two peaks were observed in the chromatography. Selected fractions from these peaks were screened for antibody activity by indirect ELISA method. Purity of the peaks were evaluated by Sodium Dodecyl Sulfate Polyacrylamide Gel Electrophoresis (SDS-PAGE) and western blotting.

For SDS-PAGE analysis, protein samples from chromatographic peaks were denatured with β-mercaptoethanol and loaded on discontinuous gel system with 5% (w/v) polyacrylamide loading gel and 12 % (w/v) separating gel, with the methods obvious to the skilled in the art. SDS PAGE marker composed of proteins spanning 10 to 170 kDa molecular weight (thermo Scientific 26616) was used. 0.2 μg purified BSA (Sigma, B2518) the major component of bovine serum and the major impurity in the antibody solution was loaded to the gel as control. 3,5 and 0,5 μg protein from the first and the second peak respectively, were loaded and compared. Electrophoresis was conducted with Bio-Rad mini protean tetra cell gel apparatus under 120 V continuous voltage. Gels were stained with Silver staining with the methods known to skilled in the art. For Western blot analysis, the proteins on the gels were transferred on a PVDF membrane in methanol containing transfer buffer (0.02 M Trizma base, 0.15 M Glycin, 0,0003 % SDS and 20% Methanol) using a semi-dry Western blot apparatus (Bio-Rad, Trans-Blot Turbo Transfer System) for 30 min. at 200 mA. The membrane was then blocked for 1 h in blocking-buffer containing 1× PBS, 1 % skim milk powder. For immunoblot analysis 1:5000 diluted AP labeled Rabbit anti mouse polyvalent antibody was incubated with the membrane for 1 h. After washing with % 0.2 Tween-PBS, the blots were visualized on the membrane with AP substrate (Sigma B5655).

**Example 9: Solvent tolerance of D12E2 antibody**

ELISA plates were coated with 500 ng Aflatoxin B1-BSA conjugate and blocked with 1 % skimmed milk solution. 0%, 10%, 20%, 30%, 40%, 50%, 60% and 70% aqueous solutions of methanol, acetonitrile, acetone and ethanol was prepared. Ammonium sulfate precipitated D12E2 antibody was 500 fold diluted with these solutions and incubated at room temperature for 5 minutes. 100 μL of the antibody solutions in differing solvent concentrations were loaded to the designated wells of the ELISA plate and incubated for 20 min. at room temperature. Wells were washed thoroughly and 2000 fold PBS diluted AP conjugated rabbit anti mouse polyvalent antibody was dispensed 100 μL/well. ELISA plate was incubated at 37°C for 1 hour and wells were washed. 100μL of 1mg/mL AP substrate 4-nitrophenyl phosphate dissolved in substrate buffer was added to the wells and incubated for 60 min. at room temperature. Absorbance at 405 nm was measured with a microplate reader.

**Example 10. Acid and Salt tolerance of the antibody**

ELISA plates were coated with 500 ng Aflatoxin B1-BSA conjugate and blocked with 1 % skimmed milk solution. 100 μL 1000 fold diluted ammonium sulfate precipitate of the D12E2 antibody was incubated on the wells for 1 h. at room temperature. 0,1 M Glycine HCl buffer at pH:7, 6, 4 and 2 were prepared. 0,1 M glycine buffer at pH:6 containing 0, 300 mM, 50 mM and 1000 mM NaCl was prepared. 100 μL of each prepared buffer was dispensed to designated wells of the ELISA plate and incubated on the wells for 5, 10 or 20 minutes at room temperature. Wells were washed and antibodies bound to the wells after the application were quantified by visualization with AP conjugated secondary antibody. 5000 fold diluted AP conjugated rabbit anti mouse polyvalent antibody was dispensed 100 μL/well. ELISA plate was incubated at 37°C for 1 hour and wells were washed. 100μL of 1mg/mL AP substrate 4-nitrophenyl phosphate dissolved in substrate buffer was added to the wells and incubated for 60 min. at room temperature. Absorbance at 405 nm was measured with a microplate reader.

**Example 11: Matrix interference effect assessment by Indirect competitive ELISA**

ELISA plates were coated with 500 ng Aflatoxin B1-BSA conjugate and blocked with 1 % skimmed milk solution. 1000 fold diluted ammonium sulfate precipitated D12E2 was prepared in PBS. 1 mL of diluted antibody was preincubated with 0, 1, 5, 10, 50 and 100 $\mu$L of 91 ppb aflatoxinB1 spiked corn, red pepper and hazelnut extracts and 0, 0.01, 0.1, 1, 5 and 10 ng aflatoxin B1 in PBS for 30 min at 37°C. 100 $\mu$L from each incubation was added to the wells and incubated for 1 hour at room temperature. Wells were washed thoroughly and2000 fold PBS diluted AP conjugated rabbit anti mouse polyvalent antibody was dispensed 100 $\mu$L/well. ELISA plate was incubated at 37°C for 1 hour and wells were washed. 100$\mu$L of 1mg/mL AP substrate 4-nitrophenyl phosphate dissolved in substrate buffer was added to the wells and incubated for 60 min. at room temperature. Absorbance at 405 nm was measured with a microplate reader.

**Example 12: Immunoaffinity column preparation**

CnBr activated sepharose 4B (Sigma C9142) was activated in acidic pH. Ammonium sulfate precipitate of the D12E2 antibody was 10 times diluted with binding buffer (0,1 M NaHCO, 0,5 M NaCl, pH 8,3). 1 mL of diluted antibody solution was used to bind 1 mL sepharose. The unbound moieties on the sepharose was blocked with 1 M Ethanolamine, pH: 8.400 $\mu$L resin was used per column.

Column performance was evaluated with overflow and limit binding tests. Both test results were evaluated by HPLC analysis. CoBrA cell method was employed for the derivatization of the aflatoxins. 20 $\mu$L sample was loaded to 250/4,6Nucleosil 100-5 C18 column (Macherey-Nagel 720014.46) at 1 mL/min flow rate at room temperature. 55% KBr-$HNO_2$ buffer + 27% methanol + 18% acetonitrile was used as mobile phase. Toxins were detected with Flourescence detector at 360 nm excitation and 430 nm emission wavelength. Device was calibrated with certified aflatoxin standards for accurate quantification.

For the overflow test 20% methanol-water or 20% methanol containing corn extract was contaminated with 25 ppb aflatoxin B1, B2, G1 or G2. 20 mL of each solution was loaded to the columns at 3 mL/min flow rate and the columns were washed with 20 mL distilled water at the same flow rate. The bound aflatoxin was eluted with 1 mL methanol. Aflatoxin concentration was calculated with HPLC analysis using 1:1 dilution of the eluate with distilled water.

The limit binding test was conducted by contaminating 16% methanol-water solution with 0,25ppb aflatoxin B1, B2, G1 or G2 each. 20 mL of the contaminated mixture was loaded to the columns at 3 mL/min flow rate and the columns were washed with 20 mL distilled water at the same flow rate. The bound aflatoxin was eluted with 1 mL methanol. Aflatoxin concentration was calculated with HPLC analysis using 1:1 dilution of the eluate with distilled water.

**Example 13: ELISA Test System**

Aflatoxin B1-BSA was conjugated to horse radish peroxidase (HRP) using (gluteraldehyde method) the methods known to the skilled in the art. 700 ng of purified antibody was coated to each ELISA plate well in PBS by overnight incubation at +4°C. The wells were washed three times with % 0.2 Tween-PBS and blocked with 400 $\mu$L 1% skimmed milk solution in PBS for 2 hours at room temperature. 400 ng aflatoxin B1-BSA-HRP conjugate was mixed with varying concentrations of unconjugated aflatoxin in %17,5 Methanol, %25 hazelnut, red pepper and corn extract. To eliminate background signal, 0,1 M $KPO_4$, 1 M NaCl pH 8 buffer is used for dilutions in the quantification of aflatoxin B1 in red pepper extract. PBS is used for dilution of other extracts. Competition with clean extract was used as negative control. Every sample/standard is studied twice. After 20 min of incubation at room temperature, wells were washed five times with % 0.2 Tween-PBS. TMB HRP substrate was added to the wells and incubated for 20 min. The reaction was stopped after incubation with 2 M $H_2SO_4$ Absorbance at 450 nm was measured with a microplate reader.

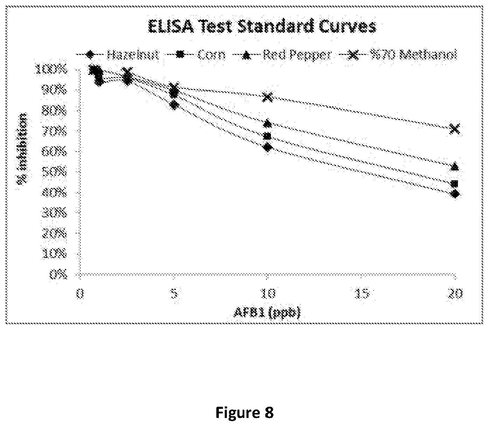

The standard curve was plotted by using average % values (Figure 8).

**Claims**

1. Use of murine monoclonal antibody of IgA class, wherein said antibody comprises a heavy chain polypeptide having the sequence of SEQ ID NO:I and a light chain polypeptide having the sequence of SEQ ID NO:2, in an immunoassay for detecting and/or quantifying aflatoxins B1, B2, G1, G2 and M1.

2. The use according claim 1 wherein said immunoassay is of the type selected from the group of immunoaffinity coloumn, enzyme immunoassay, immunochromatographic strip assay, agglutination test and an immunobiosensor assay.

3. The use according to claim 1 wherein said antibody is of a murine monoclonal antibody of IgA class or an aflatoxin binding fragment thereof.

4. The use of according to claim 1 wherein said antibody is tolerant to 40% (w/w) methanol, 20% (w/w) acetonitrile, 30% (w/w) acetone and 40% (w/w) ethanol.

5. An antibody of IgA class or an aflatoxin binding fragment thereof comprising SEQ ID NO:1 and SEQ ID NO:2.

6. An antibody according to claim 5, wherein said antibody comprises a heavy chain polypeptide having the sequence of SEQ ID NO:I and a light chain polypeptide having the sequence of SEQ ID NO:2.

7. Use of the antibody according to claim 5 or 6 detecting and/or quantifying of an aflatoxin B1, B2, G1, G2 and M1 in foods.

8. Use according to claim 7 wherein the food comprises a corn, red pepper or hazelnut matrix.

9. Use of murine monoclonal antibody of IgA class comprising SEQ ID NO:1 and SEQ ID NO:2. or an aflatoxin binding fragment thereof in an immunoassay for detecting and/or quantifying aflatoxins B1, B2, G1, G2 and M1.

**Patentansprüche**

1. Verwendung eines monoklonalen Murinantikörpers der IgA-Klasse, wobei der Antikörper ein Polypeptid der schweren Kette mit der Sequenz von SEQ ID NO:1 und ein Polypeptid der leichten Kette mit der Sequenz von SEQ ID NO:2 umfasst, in einem Immunoassay zum Nachweis und/oder zur Quantifizierung der Aflatoxine B1, B2, G1, G2 und M1.

2. Verwendung nach Anspruch 1, wobei der Immunoassay von dem Typ ist, der ausgewählt ist aus der Gruppe der Immunaffinitätssäule, des Enzymimmunoassays, des immunochromatographischen Streifenassays, des Agglutinationsassays und eines Immunobiosensorassays.

3. Verwendung nach Anspruch 1, wobei der Antikörper aus einem monoklonalen Murinantikörper der IgA-Klasse oder einem Aflatoxin-bindenden Fragment davon besteht.

4. Verwendung eines Antikörpers nach Anspruch 1, wobei der Antikörper tolerant gegenüber 40 Gew.-% Methanol, 20 Gew.-% Acetonitril, 30 Gew.-% Aceton und 40 Gew.-% Ethanol ist.

5. Antikörper der IgA-Klasse oder ein Aflatoxin-bindenden Fragment davon bestehend aus SEQ ID NO:1 und SEQ ID NO:2.

6. Antikörper nach Anspruch 5, wobei der Antikörper ein Polypeptid der schweren Kette mit der Sequenz von SEQ ID NO:1 und ein Polypeptid der leichten Kette mit der Sequenz von SEQ ID NO:2 umfasst.

7. Verwendung des Antikörpers nach Anspruch 5 oder 6 zum Nachweis und/oder zur Quantifizierung eines Aflatoxins B1, B2, G1, G2 und M1 in Lebensmitteln.

8. Verwendung nach Anspruch 7, wobei das Lebensmittel eine Mais-, Paprika- oder Haselnussmatrix umfasst.

9. Verwendung des monoklonalen Murinantikörpers der IgA-Klasse, umfassend SEQ ID NO:1 und SEQ ID NO:2 oder ein Aflatoxin-bindenden Fragment davon in einem Immunoassay zum Nachweis und/oder zur Quantifizierung der Aflatoxine B1, B2, G1, G2 und M1.

**Revendications**

1. Utilisation d'un anticorps monoclonal murin de classe IgA, dans laquelle ledit anticorps comprend un polypeptide à chaîne lourde ayant la séquence de SEQ ID NO:I et un polypeptide à chaîne légère ayant la séquence de SEQ ID NO:2, dans un dosage immunologique pour la détection et/ou la quantification des aflatoxines B1, B2, G1, G2 et M1.

2. Utilisation selon la revendication 1 dans laquelle ledit dosage immunologique est du type choisi dans le groupe constitué par la colonne d'immunoaffinité, le dosage immunoenzymatique, le dosage immunochromatographique sur bandelette, le test d'agglutination et le dosage immunobiocapteur.

3. Utilisation selon la revendication 1 dans laquelle ledit anticorps est un anticorps monoclonal murin de classe IgA ou un fragment de liaison à l'aflatoxine de celui-ci.

4. Utilisation selon la revendication 1 dans laquelle ledit anticorps est tolérant à 40% (p/p) de méthanol, 20% (p/p) d'acétonitrile, 30% (p/p) d'acétone et 40% (p/p) d'éthanol.

5. Anticorps de classe IgA ou un fragment de liaison à l'aflatoxine de celui-ci comprenant SEQ ID NO:1 et SEQ ID NO:2.

6. Anticorps selon la revendication 5, dans lequel ledit anticorps comprend un polypeptide à chaîne lourde ayant la séquence de SEQ ID NO:I et un polypeptide à chaîne légère ayant la séquence de SEQ ID NO:2.

7. Utilisation de l'anticorps selon la revendication 5 ou 6 détectant et/ou quantifiant une aflatoxine B1, B2, G1, G2 et M1 dans les aliments.

8. Utilisation selon la revendication 7 dans laquelle l'aliment comprenant une matrice de maïs, de poivron rouge ou de noisette.

9. Utilisation d'un anticorps monoclonal murin de classe IgA comprenant SEQ ID NO:1 et SEQ ID NO:2 ou un fragment de liaison à l'aflatoxine de celui-ci dans un dosage immunologique pour la détection et/ou la quantification des aflatoxines B1, B2, G1, G2 et M1.

**Figure 1**

**Figure 2**

**Figure 3A**

**Figure 3B**

**Figure 4**

**Figure 5**

**Figure 6**

**Figure 7**

**Figure 8**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4791067 A
- US 5534411 A
- US 20100062542 A1
- US 8153767 B2
- US 20140057294 A1
- US 4818687 A
- US 6194552 B
- US 20030203412 A1
- EP 2090590 A1

### Non-patent literature cited in the description

- **MURPHY et al.** *Garland Science,* 2012
- **KENSLER et al.** *Toxicological Sciences,* 2010, vol. 120 (1), S28-S48
- **SHEPHARD.** *Analytical and bioanalytical chemistry,* 2009, vol. 395 (5), 1215-1224
- **ROBENS et al.** *Reviews of environmental contamination and toxicology,* 1992, vol. 127, 69
- **BOUTRIF.** *Natural toxins,* 1995, vol. 3 (4), 322-326
- *Official Journal of the European Communities L.,* 1998, vol. 201, 436
- **UCHIGASHIMA et al.** *Journal of agricultural and food chemistry,* 2009, vol. 57 (19), 8728-8734
- **BRUCK et al.** *Journal of Immunological Methods,* 1982, vol. 53, 313-319
- **ZHOU et al.** *Journal of Immunological Methods,* 2007, vol. 328 (1-2), 79-88

SEQUENCE LISTING

      <110> TUBITAK özturk, Selma; Ertekin, Özlem; Pirinçci, þerife þpeyda; Kocaaða, Harun

      <120> MONOCLONAL IGA ANTIBODY FOR MYCOTOXINS, A HYBRIDOMA CELL LINE PRODUCING SAID ANTIBODY, AND SPECIFIC USE THEREOF IN IMMUNOASSAYS

      <130> TUBITAK

      <160> 2

<170> PatentIn version 3.5

<210> 1
<211> 390
<212> DNA
<213> Mus musculus

<400> 1

```
ggagatggtg ggatttctcg cagactctga ggagacggtg accgtggtcc ctgcgcccca        60

gacatcgaag taccagtagc cccagttggg tctagcacag taatagacgg cagtgtcctc       120

agatgtcagg ctgctgagct gcaggtaggc tgtgttggag gaagtgtctg cagtgacagt       180

ggccttgtcc tggaaattct ggacatataa agtatgacca ttcgcaggat caatccttcc       240

aatccactcc aggccctgtt caggcctctg cttcacccag tgaatatagt tgtctctaat       300

gttaaagcca gaagctgtgc aggacaactt gactgaggcc ccaggcctca caagctcagc       360

ccctgactcc tgcagcttca cctccggaag                                        390
```

<210> 2
<211> 350
<212> DNA
<213> Mus musculus

<400> 2

```
ggtggataca gttggtgcag catcagcccg tttcagctcc agcttggtcc cagcacccaa        60

cgtgaggcca gctgttactc tgttgacaga aatacattcc aaaatcttca gtctccacac       120

tgttgatact gagagtgaaa tatgtccctg atccactgcc actgaacctg gaggggatcc       180

cagagatgga ctgggaagca aacttcatga gaagccttgg agactcatgt gatttttgtt       240

gataccagtg taggaagttg ctaatacttt gactggccct gcaggaaaga ctgactctat       300

ctcctggagt catagacagg gtggctggag tctgggtcat cacaatgtcc                  350
```